# EUROPEAN PATENT SPECIFICATION

(11) **EP 3 096 679 B1**
(45) Date of publication and mention of the grant of the patent: **11.10.2023**
(21) Application number: 15700597.6
(22) Date of filing: 20.01.2015
(51) Int. Cl.: A61B 5/00, A61B 5/103, A61F 13/00, G01N 21/80, A61B 5/145, A61F 13/42

(54) **SYSTEMS AND METHODS FOR WOUND MONITORING**
SYSTEME UND VERFAHREN ZUR WUNDÜBERWACHUNG
SYSTÈMES ET MÉTHODES DE SURVEILLANCE DE PLAIE

(30) Priority: 23.01.2014 GB 201401112
(43) Date of publication of application: 30.11.2016
(73) Proprietor: Smith & Nephew plc, Watford, Hertfordshire WD18 8YE (GB)
(72) Inventor: SAXBY, Carl, Heslington York YO10 5DF (GB); HICKS, John, Kenneth, Heslington York YO10 5DF (GB); HAMMOND, Victoria, Jody, Heslington York YO10 5DF (GB); CUVELIER, Sebastien, Antoine, Yves, Bumpstead Haverhill CB9 7DG (GB); WICKS, Benjamin, Histon Cambridge CB24 9JN (GB)
(74) Representative: Smith & Nephew
(86) International application number: PCT/EP2015/050964
(87) International publication number: WO 2015/110411

(56) References cited:
- EP-A1- 0 430 608
- WO-A1-2008/125995
- WO-A1-2012/074509
- CN-Y- 201 414 880
- GB-A- 2 408 330
- JP-A- 2007 163 350
- US-A1- 2005 105 789
- US-A1- 2012 201 437
- US-A1- 2013 303 865
- B.Y. Loh ET AL: "Automated Mobile pH Reader on a Camera Phone", IAENG International Journal of Computer Science, 1 August 2011 (2011-08-01), pages 268-274, XP055213411, Retrieved from the Internet: URL:http://doaj.org/search?source=%7B%22qu ery%22%3A%7B%22bool%22%3A%7B%22must%22%3A% 5B%7B%22term%22%3A%7B%22id%22%3A%22806777d 5025148db9fc2873f8086d4ac%22%7D%7D%5D%7D%7 D%7D

## Description

### Background

Wound treatment often involves monitoring a wound during healing for indications of the status and progress of the wound. Monitoring indications of wound health can indicate the efficacy of delivered treatment or signal to a physician a need for a change in treatment. One indicator in particular that is useful for this monitoring is the pH level of the wound tissue. The pH of the tissue can indicate or affect a number of factors relevant to wound healing, such as oxygen release, angiogenesis, protease activity, and bacterial toxicity. For example, wounds having elevated alkaline pH levels have been shown to have lower rates of healing than wounds in which the pH is closer to or below a neutral 7.0 pH level. For example, if a chronic wound has a pH between 6 and 7.5, this often indicates that wound healing is progressing well and treatment is working. If the pH rises to between 7.5 and 8.5, this can be an indication that the wound should be monitored and treatment adjusted to lower the pH level. By monitoring this pH level over the course of the wound healing, a physician may be better able to assess whether healing is progressing well or whether intervention or a change in treatment is needed.

WO2008125995 discloses a system for concurrently capturing vital sign data and an image of a patient includes a vital signs monitor that receives patient parameter data and image data, and an imager, coupled to the VSM, that captures an image of a patient and transmits image data to the VSM. EP0430608 discloses a dressing having a skin or wound contacting surface that is provided with an indicator means on the opposite surface. GB2408330 discloses a method of assessing the pH of a substrate or environment, the method comprising contacting the substrate with a test material or introducing the test material into an environment, wherein said test material is arranged to change colour according to pH. JP2007163350 relates to an observation device that includes an imaging device that acquires a color image of an object to be observed dyed with a pH indicator. US2005105789 relates to a method and apparatus for locating changes that take place over time in a visual image and particularly to a computerized method for digitally processing temporal images to enhance potentially minute changes in the image. WO2012074509 discloses patch based sensors to provide a panel of specific analyte parameters that determine one or more physiological conditions and/or the level of healing progression of a wound. B.Y. Loh et al. "Automated Mobile pH Reader on a Camera Phone", |AENG International Journal of Computer Science, 24 August 2011 on pages 268-274 discloses an algorithm implemented on a cameral phone that automatically identifies the pH level on a test strip.

Direct measurement of wound pH levels, for example using a pH probe or applying a color sensitive pH strip, may be unsuitable for pH monitoring during wound healing. The use of a probe or strip may disrupt or irritate the wound and hamper wound healing. To facilitate wound monitoring, a pH- sensitive bandage may be provided that changes color as the pH of the wound changes. These bandages can be a quick and easy indication to a patient or a physician of the pH of the wound to which the bandage is applied, and the changing color of the wound dressing can provide a signal that the pH of that wound is changing. While these bandages provide helpful indications, interpretation of the color indicators is reliant on the subjective assessment of the patient or physician. Often that subjective judgment involves comparing the color of the dressing to a standard color strip or scale and estimating where on the scale the indicated pH falls. As a result, the assessment and judgment is often limited in accuracy and resolution. The color determination may also be hampered by differences in color perception
between individuals, differences in lighting conditions when a bandage is assessed, color blindness, or other conditions that affect color perception.

### Summary

The scope of the invention is defined by the appended set of claims. Disclosed herein are systems for wound monitoring, and in particular for monitoring wound pH levels to assess the efficacy and need for intervention in wound treatment. The approaches described provide a system and method for determining and monitoring changes in a wound pH level and applying the wound pH information to determine any needed changes in treatment. These systems and methods, when used in combination with pH indicative wound dressings, can provide a quick and accurate indication or assessment to a physician or a patient of current wound status and treatment progression.

The embodiments described herein automate the calculation of a pH value from a bandage color. This reduces the subjectivity of the bandage reading and reduces the variance in readings that can result from that subjectivity. After a physician, patient, or other user takes a photo of the bandage, the image is processed to determine the indicated pH using a computer-implemented process. This process analyzes all users' images, thus reducing the variation caused by color blindness or other differences in color perception between individuals. The image processing can also correct for lighting or image quality differences between readings, thus improving accuracy compared to the subjective human determinations. The image processing thus provides accurate and reliable pH readings across a variety of conditions. Because the bandage itself indicates the pH, these helpful readings can be taken without moving the wound dressing. As a result, the risk of infection and hampering wound healing is reduced compared to manual inspections of the wound directly.

### Brief Description of the Drawings

The foregoing and other objects and advantages will be apparent upon consideration of the following detailed description, taken in conjunction with the accompanying drawings, in which like reference characters refer to like parts throughout.
Figure 1 shows a flow chart for a method of monitoring wound pH levels according to some implementations.
Figure 2 shows a flow chart for a method of processing a wound dressing image and calculating a pH level based on the color of the image according to some implementations.
Figure 3 shows a visual representation of a pH calculation process according to some implementations.
Figure 4 shows an illustrative pH-sensitive wound dressing according to some implementations.
Figure 5 shows an illustrative system including a user device, a server, and a communications network according to some implementations.
Figure 6 shows an illustrative user device according to some implementations.
Figure 7 shows an illustrative computing device according to some implementations.
Figures 8-13 show illustrative user device screenshots according to some implementations.

### Detailed Description

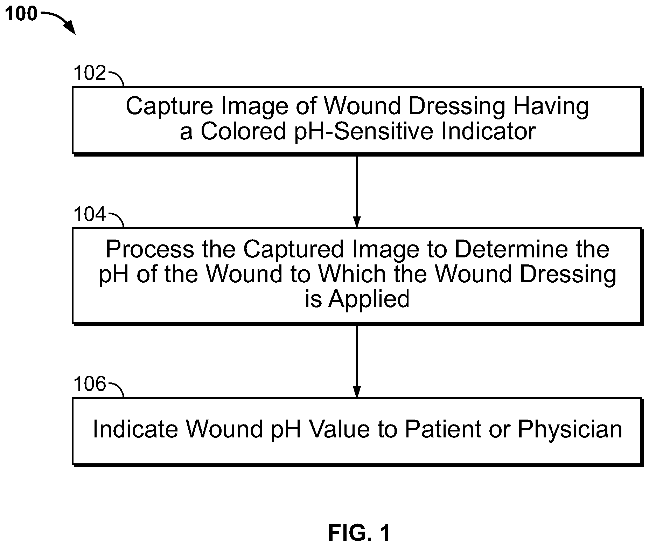

An automated wound dressing image processing system provides a physician or patient with an effective and reliance approach to monitoring a wound, such as when negative pressure wound therapy is applied to the wound. Figure 1 shows a wound monitoring method 100 that informs the patient or physician of wound status indicated by a color-coded pH indicator wound dressing. The method 100 automates the determination of bandage color to provide accurate readings of wound pH. Rather than relying on a user's judgment in discerning the color of a wound pH indicator and relating the discerned color to a pH scale, the method 100 allows the user to capture a digital image of the wound dressing and applies image processing techniques to determine the indicated pH. The resulting monitoring method thus improves the reliability of pH readings that may be used to apply or change the treatment regimen applied to the healing wound. The illustrated method can be implemented by a a user device alone or in combination with one or more computing devices, such as a server.

The wound monitoring method 100 begins when an image of a color-coded wound pH indicator is captured at step 102. The indicator is disposed on a patient's wound dressing and includes one or more pH-sensitive dyes or compounds that change color as the pH of the wound to which the dressing is applied changes. For example, a particular dye may exhibit a spectrum of colors, from lighter yellow and orange colors to darker red or purple colors, when exposed to different pH values over the range from 0 to 14. Particular dyes and compounds disposed on such bandages may vary, and any suitable wound pH indicators, exhibiting any suitable known spectrum of colors, may be employed in the systems and methods of the present disclosure.

The image captured at step 102 of method 100 is taken on a user device, for example, by a physician during a patient visit or by the patient away from the doctor's office. With the advancements in smartphones and mobile technology, the range of user devices suitable for use in method 100 is quite broad. Any user device having a camera for image capture and circuitry either to process the image locally or transmit the image for remote processing is suitable for the method. For example, smartphones, tablet computers, laptop computers, digital cameras, web-enabled cameras, or any other user devices with image capturing and processing or communication circuitry could be used at step 102. The portability of many of these devices provides an advantage by allowing the user to capture the image at step 102 in virtually any location with the user device. Thus, the method 100 provides a patient with wound monitoring and feedback without requiring constant check-ups with a doctor or visits to the doctor's office to obtain a reading.

The image captured at step 102 is processed at step 104 to determine the pH of the wound that is indicated by the color of the wound dressing. In some embodiments, the image is captured on a mobile device, for example using an app on a smartphone, and the mobile device itself performs the processing at step 104 to calculate the indicated pH. In other embodiments, the user device transmits the captured image to a remote location, for example to a server, where the image is processed to calculate the pH. The server may then transmit the calculated pH back to the user device for display and use in evaluating the wound status. The server, the user device, or both can also store a record of the pH readings for trend and progress analyses.

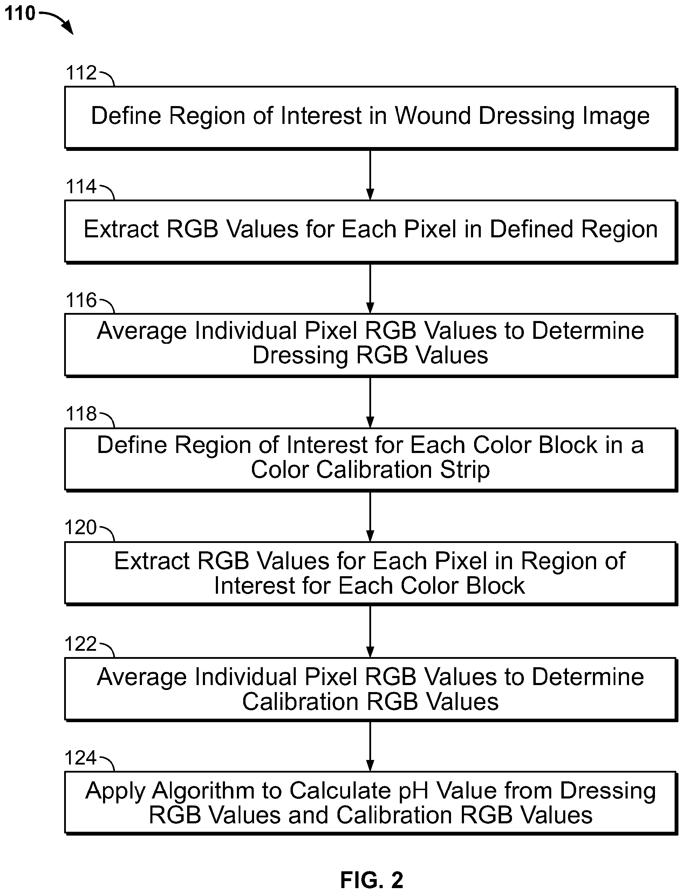

The image processing at step 104 automatically characterizes the color of the wound dressing in the captured image. This may be done, for example, by determining where the color of the dressing lies in the RGB color model (or any other suitable color model such as CMYK, Lab colour space, and the like). The processing applied to the image determines the red, blue, and green components of the overall dressing color and uses the relative presence of the three colors to characterize the color. The RGB characterization values are then compared to standardized pH RGB values to calculate the pH indicated by the wound dressing. Further details on this processing and pH calculation are discussed below, for example in relation to Figure 2.

After the pH of the wound is determined at step 104, the calculated pH value is relayed to the patient or physician at step 106. The pH is displayed either on the user device used to capture the image or on another device in communication with the user device or with a server in the system. In addition to the calculated value, the pH may be displayed at step 106 with one or more accessory features, such as a graph showing past pH readings for the patient, a list of pH readings, an indication of wound health based on the reading, a suggested mode of treatment for the wound, or any other suitable information for the patient or physician. In addition, the display may provide the user with various options for storing or identifying the pH reading. For example, step 106 may include displaying an option to the user to accept or reject the pH reading, and may prompt the user to capture a new image if the pH reading is rejected. The display may also provide the user with an option to identify the patient for record keeping, where the calculated pH value is associated with either an already stored patient or a newly identified patient for tracking the progress of the wound. In some embodiments, the patient may be automatically identified by a barcode or a QR code on the bandage in the image.

The method 100 provides prompt feedback and an accurate pH reading, by implementing automated image processing and analysis at step 104. The color extraction and pH calculation performed at step 104 can provide improved accuracy in the pH reading, and thus in the treatment decisions that are based on the pH. The processing applied to the image first automates the determination of wound dressing color, and then applies processing to provide a pH read out from the detected color.

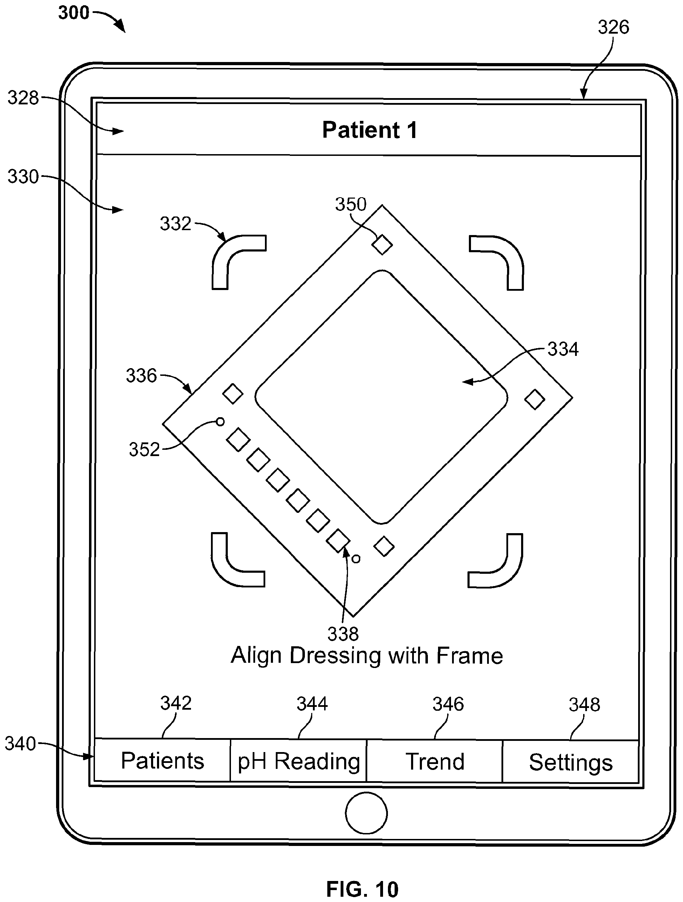

Figure 2 shows a method 110 for processing a wound dressing image to provide a patient or physician with a pH readout, which may be the method performed at step 104 in Figure 1. The illustrated method can be implemented by a user device alone or in combination with one or more computing devices, such as a server. After an image is captured or received, system implementing the method 110 defines or identifies a region of interest in the wound dressing captured in the image at step 112. The region of interest defines the area of the image that is determined to be an adequate representation of the wound dressing for color and pH determinations. The region may have any suitable shape and size, for example a circle or a square having a radius or area defined by a predetermined number of pixels. In some embodiments, the region is defined as the pixels that form a particular shape of a particular size around a determined center point of the wound dressing image. In such embodiments, the center of the dressing image is first defined at step 112, and the region of interest is defined based on the shape and size settings around the defined center point. A circular region around the center point can be defined having a radius of any suitable number of pixels, for example five pixels, ten pixels, twenty pixels, fifty pixels, one hundred pixels, or any other suitable radius. The shape and size of the region may be an oval, rectangle, square, triangle, trapezoid, or any suitable shape of a suitable size. The shape and size of the region may also be defined based on the resolution settings of the camera used to capture the image, or on the size of the captured image. In certain implementations, the method 110 defines or identifies multiple regions of interest. Any suitable number and dimensions of the multiple regions can be selected. For example, the multiple regions can be five circules as depicted in Figure 10 (namely one circular region in the middle or the dressing surrouned by four circular regions in each of the four corners of the dressing).

When the region of interest for analysis has been defined, a system implementing the method 110 characterizes the color of the dressing, as determined from the region of interest, at step 114. Each pixel lying within the defined region of interest is analyzed to extract the RGB values for the pixel color from the image. Particularly for cases in which the region of interest is larger, there may be variation of the exact color of the wound dressing over the pixels included in the region. Thus, it may be preferable to define the region of interest large enough to capture pixels that will provide an accurate representation of the dressing color. Including too few pixels in the region may result in one pixel or small area of the dressing, for example a pixel or area that is artificially dark due to poor imaging, skewing the overall RGB characterization and affecting the accuracy of the pH value calculated from the characterization. If the region is defined broadly enough, the extraction of RGB values at step 114 will include enough accurate pixel readings to dilute the effect of any artificial pixels or areas. Alternatively, a filter may be applied to remove these artificially light or dark pixels from the analysis. For example, individual pixels that exhibit RGB values that lie a certain distance from the average RGB values of the pixels in the region of interest may be designated as outliers and removed from the analysis.

After the individual pixel RGB values within the region of interest are extracted from the image, the values are averaged at step 116 to determine the RGB value characterization for the overall dressing in the image. By averaging the pixel values at step 116, the method 110 provides an accurate automated reading of the color of the dressing, as determined by the color or colors present within the defined region of interest. The averaged RGB values determined at step 116 are the values that are then used in calculating a pH value indicated by the imaged wound dressing indicator.

In order to provide accurate pH calculations from the values determined at step 116, the method 110 includes performing color determinations for calibration color squares. The calibration colors can provide readings of standardized colors that are used to normalize the dressing RGB values. This normalization can account for variation in captured images, for example caused by variations in the image capture device, positioning of the wound dressing, lighting when the image is captured, type of wound dressing, pH-sensitive dye included in the dressing, or other factors that may affect captured dressing images. The calibration colors that are analyzed may be included in the same image as the dressing image, for example as a strip provided on the dressing itself or a strip placed in this image window with the dressing, or may be captured in a separate image under the same or similar conditions as the dressing image. Providing the strip on the dressing itself may be convenient for the user as a separate strip does not need to be included in each image. Using a separate strip may also be advantageous for reproducing, for example, for a doctor who uses the same strip for all patients. The separate strip may also ensure the color blocks do not become discolored from blood in the wound under the bandage.

The calibration colors that are captured for normalization exhibit the expected color of the wound dressing at a range of pH values. The colors may be provided as a series of color blocks, for example as three, five, seven, or more blocks, in a strip on the wound dressing. The color blocks may show the expected colors at set pH increments, for example at one or more of pH values 4.0, 5.0, 6.0, 7.0, 8.0, 9.0, intermediate values between those pH values, or pH values above 9.0 or below 4.0. Whether these color blocks are included as a color calibration strip on the wound dressing or as a separate component by an image, the standardized colors are processed beginning at step 118 of method 110 to normalize the dressing RGB values determined at step 116 and reduce the effect of image variation.

At step 118, similar to step 112 for the dressing, a region of interest is defined for each calibration color block included in the captured color calibration strip. As with the dressing, each region of interest may be defined as a set shape of a set number of pixels surrounding the center of the color block. The shape and size of each color block region of interest may be the same as the shape and size of the dressing region of interest, or may be a different shape or size. For example, in an embodiment where the color calibration strip is provided at the bottom of the wound dressing, the color blocks may be smaller than the dressing area, and the regions of interest may also be defined as smaller pixel areas.

The defined region of interest in each color block is analyzed at step 120 to extract RGB value characterizations of the calibration colors. Though the colors in the strip may be the same between different dressings or different images of the same dressings, variations in lighting and image capture condition can cause different RGB value determinations at step 120 between readings. The purpose of the color calibration strip is to identify, and correct for, this variation in readings of identical colors between images. As with the dressing RGB values, the individual pixel RGB values extracted for each color block at step 120 are averaged at step 122 to provide a single set of RGB values for each standardized color block in the calibration strip.

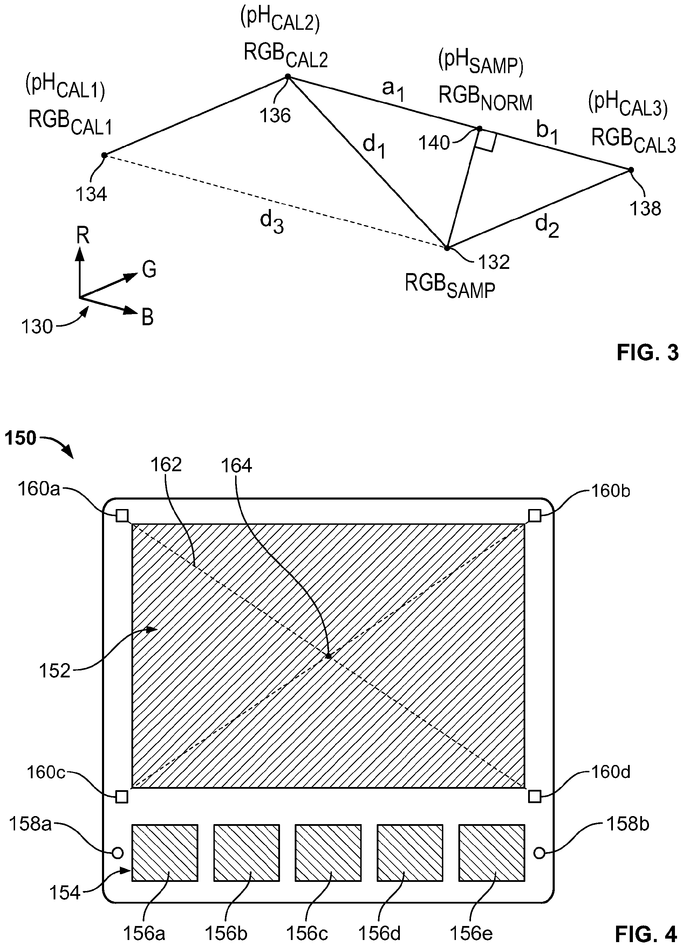

After the individual calibration pixel RGB values are averaged at step 122, the image has been processed to determine a first set of RGB values that characterizes the dressing color and a series of RGB value sets, one for each color block, each of which characterizes a standardized color in the color calibration strip. The dressing RGB values and the calibration RGB values are combined at step 124, and a single pH value for the wound dressing is calculated and provided to the user. While the exact processing that is applied at step 124 may vary, an illustrative process is shown visually in Figure 3.

Figure 3 depicts a process that treats each RGB value in an extracted wound dressing RGB value set and three extracted calibration RGB value sets as point locations in a three-dimensional space represented by RGB axis 130. The illustrated process can be implemented by a user device alone or in combination with one or more computing devices, such as a server. The dressing RGB value set is depicted as point 132, labeled RGB_{SAMP}, in the three-dimensional space. The three calibration RGB value sets are depicted as points 134, 136, and 138, labeled RGB_{CAL1}, RGB_{CAL2}, and RGB_{CAL3}, respectively. While three calibration RGB points are shown in Figure 3 for illustration, any suitable number of calibration points, corresponding to the number of color blocks in the imaged color calibration strip, may be used to determine pH value.

From the plotted points, the process determines the distances between the dressing RGB point 132 and each of the calibration RGB points 134 (d₃), 136 (d₁), and 138 (d₂). These distances graphically represent the similarity between the color of the wound dressing, as defined by the RGB_{SAMP} values, and each of the calibration colors, represented by the RGB_{CAL} values. A relatively small distance between the dressing RGB values and a given set of calibration RGB values indicates similarity between the dressing and particular calibration color, while relatively larger distances indicate different colors. For example, in Figure 3, each of d₁ and d₂ are shorter than distance d₃. This indicates that the color of the wound dressing from which the RGB_{SAMP} values were extracted is more similar to the color of the color blocks from which the RGB_{CAL2} and RGB_{CAL3} values were extracted than it is to the color of the color block from which the RGB_{CAL1} values were extracted.

Once all distances between the dressing RGB value and each set of calibration RGB values are determined, the process selects the two smallest determined distances to identify the two calibration colors, and corresponding standardized pH values associated with these two calibrated colors, that are most similar to the dressing color and wound pH value. For example, in the visualization in Figure 3 distances d₁ and d₂, with corresponding RGB values RGB_{CAL2} and RGB_{CAL3}, are selected as most similar to the dressing RGB values, RGB_{SAMP}. Thus, it is determined that the pH value indicated by the colored wound dressing is closer to the standardized pH values pH_{CAL2} and pH_{CAL3} associated with RGB_{CAL2} and RGB_{CAL3}, respectively, than it is to any other standardized pH values, such as pH_{CAL1} associated with RGB_{CAL1}.

In order to calculate an estimate for the dressing pH, which falls between the selected two closest standardized pH values, the dressing RGB values are normalized to a line segment defined by the two selected calibration RGB values. In Figure 3, this line segment is shown as a line between RGB_{CAL2} and RGB_{CAL3}, which includes a point RGB_{NORM} that is the normalized RGB value for the wound dressing. The value RGB_{NORM} is defined by projecting the dressing RGB values RGB_{SAMP} perpendicularly (or in any other suitable way) onto the line defined by the two calibration RGB values. The location of the RGB_{NORM} point 140 along this line is then used to calculate the final pH estimation for the wound dressing. While selection of two calibration and smallest distances is described, the illustrated process can select less or more than two calibration valuea and/or smallest distances. In addition, in certain implementations, one or more distances other than the smallest can be utilized.

The distance between the normalized point 140 and each of the calibration RGB points 136 (distance a₁) and 138 (distance b₁) are determined and used to calculated the pH estimation. The proportions of the line between the calibration RGB points made up of these distances indicates where the pH_{SAMP} value lies between the pH_{CAL2} and pH_{CAL3} standardized values. For example, if a₁ is equal to b₁, then pH_{SAMP} is halfway between pH_{CAL2} and pH_{CAL3}. Thus, if pH_{CAL2} is 6.5 and pH_{CAL3} is 7.0, then pH_{SAMP} is calculated as 6.75. If, on the other hand, a₁ is equal to 75% of the line between RGB_{CAL2} and RGB_{CAL3}, then the pH_{SAMP} is closer to pH_{CAL3} than to pH_{CAL2}. In that case, if pH_{CAL2} is 6.5 and pH_{CAL3} is 7.0, then pH_{SAMP} is calculated as 6.875. The process returns the calculated pH_{SAMP} value for display to the user and storage in a patient record.

In some implementations, the analysis methods shown in Figures 1-3 employ a pH-sensitive wound dressing, and images captured of that dressing, to provide the user with pH readings and wound status feedback. A wound dressing 150 suitable for use in such methods is shown in Figure 4. The wound dressing 150 includes a pH indicator 152 and a color calibration strip 154. The pH indicator 152 is the pH-sensitive component of the wound dressing 150 and includes one or more dyes or compounds that exhibit different colors under different pH conditions. When the wound dressing 150 is visualized, or images of the dressing are captured, the color of the indicator 152 is used to determine a pH level for the wound to which the dressing 150 is applied.

The color calibration strip 154 is provided on the wound dressing 150 to facilitate interpretation of the indicator 152 to determine pH level. The color calibration strip 154 includes five color blocks 156a-e, each of which indicate the known color of the dye or compound in indicator 152 at a given pH level. While only five color blocks are shown on the dressing 150, more or fewer color blocks could be included on the dressing. As an alternative to providing the strip 154 directly on the dressing, the strip may be a separate component that is placed on the dressing during image capture. The color blocks 156a-e are selected to span the range of expected pH values that the dressing 150 will contact, and may be indicative of pH values spaced either at even or uneven increments over that expected range. For example, color block 156a may indicate the expected color of indicator 152 at a pH value of 5.0, and each of color block 156b-e may indicate the expected color of indicator 152 at pH values incremented by 1.0, up to a value of 9.0 for color block 156e. Other ranges and increments, varied and constant, may be used, and more or fewer than five calibration pH levels may be used for dressings having more or fewer color blocks.

The dressing 150 includes orientation indicators for automated image processing. Calibration strip location indicators 158a and 158b are provided at each end of the color calibration strip 154. These indicators 158a and 158b can be used for image processing to automatically detect the color blocks 156a-e. A processing system may locate the indicators 158a and 158b in a received image and draw a line between the two indicators. The system may then identify each of color blocks 156a-e along the drawn line. This approach may facilitate identification of the color blocks in images in which the dressing 150 is not optimally aligned, for example when the calibration color strip is not straight relative to an alignment frame in a captured image.

The dressing 150 also includes corner indicators 160a-d for automated image processing to identify the location of the pH indicator 152. As with the indicators 158a and 158b, the corner indicators 160a-d can be detected by an image processing system and used to re-orient an image of the dressing 150 that is not optimally aligned during image capture. In some implementations, it may be preferable to identify a particular region of interest within the pH indicator 152 that is used for color analysis, and the corner indicators 160a-d may be used to identify that region. For example, the image process system may define an X 162 extending between the corner indicators 160a-d. The intersection of the two lines that form the X 162 may then be processed to define center point 164 that identifies the center of the pH indicator 152. From this center point 164, the region of interest for analysis can be defined.

Various implementations of devices that are usable for the methods and wound systems described above for providing pH reading and monitoring are envisioned, including both local user devices and processing systems as well as remote server systems in communication with local devices over a network. For ease of illustration, embodiments of these devices are described below with respect to illustrative user devices, servers, and networks. The systems, devices, and methods disclosed herein, however, may be adapted to other implementations and other embodiments of such devices and networks.

As used herein, "user device" includes, without limitation, any suitable combination of one or more devices configured with hardware, firmware, and software to carry out one or more of the computerized techniques described herein. A user device can be any computing device that is capable of receiving user input, for example receiving images, and providing responsive analysis, for example providing calculated pH values or trends, to a user either as a stand-alone device or in communication with an external processing system, such as a server, over a communication network. For example, a user device may include a mobile computing device (e.g., a laptop computer, a tablet computer, a personal digital assistant (PDA), a mobile telephone (such as a smartphone), or a camera) or a stationary computing device (e.g., a personal computer, stationary telephone, or other computing device). A user device is preferably capable of wireless communications for interfacing with external systems. However, devices without wireless communication capabilities may be used without departing from the scope of this disclosure. A user device may include one or more cameras, including both front-facing and rear-facing cameras, for capturing images of wound dressings. In some implementations, a user device is a device worn by a user such as augmented reality glasses. A user device may also include software for generating or editing images.

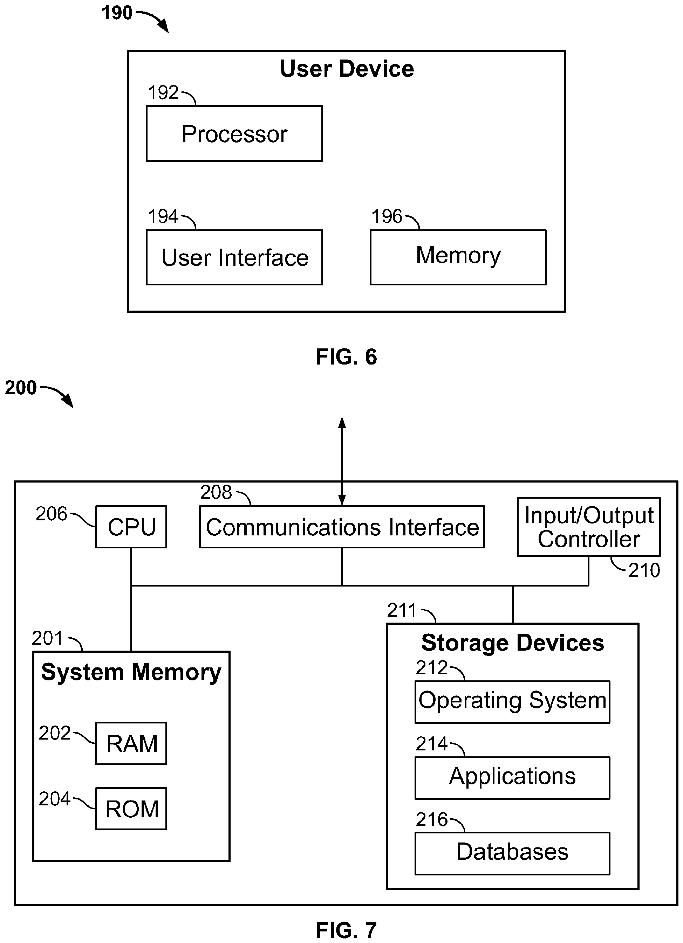

As used herein, the terms "processor," "processing circuitry," or "computing device" refers to one or more computers, microprocessors, microcontrollers, digital signal processors, programmable logic devices, field-programmable gate arrays (FPGAs), application-specific integrated circuits (ASICs), etc., and may include a multi-core processor (*e.g.,* dual-core, quad-core, hexa-core, or any suitable number of cores) or supercomputer. It may also refer to other devices configured with hardware that includes logic circuitry, firmware, and software to carry out one or more of the computerized techniques described herein. Processors and processing devices may also include one or more memory devices for storing inputs, outputs, and data that is currently being processed. An illustrative computing device, which may be used to implement any of the processing circuitry and servers described herein, is described in detail below with reference to Figure 7.

As used herein, "user interface" includes, without limitation, any suitable combination of one or more input devices (e.g., keypads, a mouse, touch screens, trackballs, voice recognition systems, gesture recognition systems, accelerometers, RFID and wireless sensors, optical sensors, solid-state compasses, gyroscopes, stylus input, joystick, etc.) and/or one or more output devices (e.g., visual displays, speakers, tactile displays, printing devices, etc.) For example, user interfaces can include a display (which may be a touch-sensitive color display, optical projection system, or other display) for graphically receiving and providing information to the user.

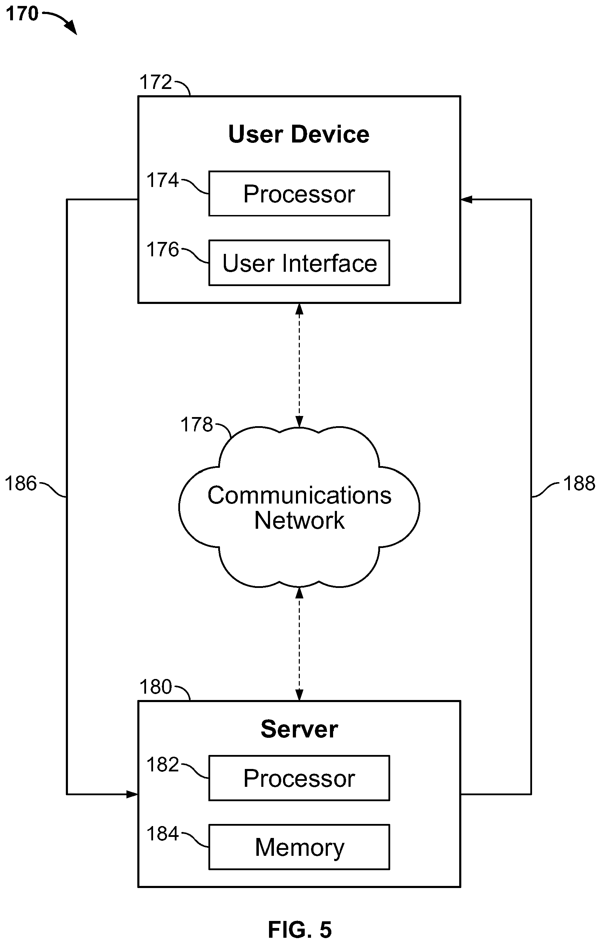

Figures 5 and 6 depict embodiments of device, a computing device, such as a server, and network structures that may be used to implement the systems and methods disclosed herein. Figure 5 is a block diagram of a computerized system 170 for providing automated reading and monitoring of wound pH status and trends. Generally, in system 170, a user device 172 and server 180 are connected over a communications network 178. The user device 172 includes processing circuitry 174 and a user interface 176. The server 180 includes processing circuitry 182 and memory 184.

During wound evaluation and monitoring, an image of a wound dressing having a pH color indicator, such as the dressing 150 discussed above, is captured by the user device 172 and transmitted to the server 180 over network 178 in transmission 186. The processing circuitry 182 at the server 180 analyzes the received image and provides feedback, for example a calculated pH value, over the network 178 in transmission 188. In addition to images and pH values, the transmission 186 and 188 may include any other information provided by the user or sent by the server 180, for example any additional user input or requests may be provided in transmission 186 and any additional information such as patient pH trends and diagnoses may be provided in transmission 188.

The network 178 couples the user device 172 and server 180 and carries transmissions, such as transmissions 186 and 188, between the two components. Communications network 178 may be any suitable network for exchanging information between user device 172 and server 180. For example, communications network 178 can include the Internet, a mobile phone network, mobile voice or data network (e.g., a 3G, 4G, or LTE network), cable network, public switched telephone network, a satellite network, or other type of communications network or combinations of communications networks. The user device 172 and server 180 can communicate using one or more communications paths, such as a satellite path, a fiber-optic path, a cable path, a path that supports Internet communications, free-space connections (e.g., for broadcast or other wireless signals), or any other suitable wired or wireless communications path or combination of such paths. The transmissions sent over the communications may be encrypted to provide secure data transmissions. The secure transmission is preferable for the sensitive patient and medical information sent by the devices.

Only one server 180 and one user device 172 are shown in Figure 5 to avoid complicating the drawing, but the system 170 can support multiple servers and multiple user devices. For example, rather than being located in the single server 180, processor 182 may be located in a first server to provide image processing and analysis, while memory 184 may be located in a second server to provide data storage and retrieval. Multiple servers may operate together as a cluster or as a distributed computing network.

In some implementations, the system 170 is implemented in a cloud computing environment in which one or more of the components are provided by different processing and storage services connected via the Internet or other communications system. In a cloud computing environment, various types of computing services for content sharing, storage, or distribution are provided by a collection of network-accessible computing and storage resources. For example, the cloud can include a collection of server computing devices, which may be located centrally or at distributed locations that provide cloud-based services to various types of users and devices connected via a network such as the Internet via communications network 178. These cloud resources may include one or more content sources and one or more data sources. In addition or in the alternative, the remote computing sites may include other user devices, such as user medical devices, user computer devices, and wireless user communications devices. For example, the other user devices may provide access to stored copies of data or images. The user devices may operate in a peer-to-peer manner without communication with the server 180. The cloud provides access to services, such as content storage, content sharing, or social networking services, among other examples, as well as access to any content described below. Services can be provided in the cloud through cloud computing service providers, or through other providers of online services. For example, the cloud-based services can include a content storage service, a content sharing site, a social networking site, or other services via which user-sourced content is distributed for viewing by others on connected devices. These cloud-based services may allow a user device to store content to the cloud and to receive content from the cloud rather than storing content locally and accessing locally-stored content. Cloud resources may be accessed by user device 172 using, for example, a web browser, a desktop application, a mobile application, and/or any combination of access applications. In some implementations, a user device receives content from multiple cloud resources simultaneously. For example, a user device can access data and information from one cloud resource while downloading or uploading content to or from a second cloud resource. A user device may also download or upload content to or from multiple cloud resources for more efficient downloading or uploading.

While Figure 5 depicts a network-based system for providing wound monitoring and wound pH determinations, the functional components of the system 170 may be implemented as one or more components included within or local to a user device. For example, Figure 6 depicts a user device 190 that includes processing circuitry 192, a user interface 194, and memory 196. The processing circuitry 194 may be configured to perform any or all of the functions of processing circuitry 174 and 182 of Figure 5, the memory 196 may be configured to store any or all of the data stored in memory 184 of Figure 5, and the user interface 194 may be configured to perform any of the input and output functions described herein for the user interface 176 of Figure 5. In some implementations, the user device 190 is configured to perform all of the functions described herein for image capture, image analysis, pH calculation, patient and pH data storage, and user interaction described herein for wound monitoring. The data stored in memory 196 can be encrypted and require password authorization for access to protect sensitive patient and medical information.

Figure 7 shows a block diagram of an illustrative computing device 200, which may be any of the computerized components of the systems in Figures 5 and 6, for performing any of the processes described herein. Each of the components of the systems 170 or 190 described in Figures 5 and 6 may be implemented on one or more computing device 200. In certain aspects, a plurality of the components of these systems may be included within one computing device 200. In certain implementations, a component and a storage device may be implemented across several computing devices 200. The computing device 200 includes at least one communications interface 208, an input/output controller 210, system memory 201, and one or more data storage device 211. The system memory 201 includes at least one random access memory (RAM 202) and at least one read-only memory (ROM 204). These elements are in communication with a central processing unit (CPU 206) to facilitate the operation of the computing device 200.

The computing device 200 may be configured in many different ways. For example, the computing device 200 may be a conventional standalone computer or alternatively, the functions of computing device 200 may be distributed across multiple computer system and architectures. In Figure 7, the computing device 200 is linked, via network or local network, to other servers or systems. The computing device 200 may be configured in a distributed architecture, wherein databases and processing circuitry is housed in separate units or locations. Some units perform primary processing functions and contain at a minimum a general controller or a processing circuitry and a system memory. In distributed architecture implementations, each of these units may be attached via the communications interface 208 to a communications hub or port (not shown) that serves as a primary communication link with other servers, client or user computers and other related devices. The communications hub or port may have minimal processing capability itself, serving primarily as a communications router. A variety of communications protocols may be part of the system, including, but not limited to, Ethernet, SAP, SAS^{™}, ATP, BLUETOOTH^{™}, GSM, DICOM and TCP/IP.

Communications interface 208 is any suitable combination of hardware, firmware, or software for exchanging information with external devices. Communications interface 208 may exchange information with external systems using one or more of a cable modem, an integrated services digital network (ISDN) modem, a digital subscriber line (DSL) modem, a telephone modem, an Ethernet card, or a wireless modem for communications with other devices, or any other suitable communications interface. Such communications may involve the Internet or any other suitable communications networks 178 as discussed in relation to Figure 5. In addition, the communications interface 208 may include circuitry that enables peer-to-peer communication, or communication between user devices in locations remote from each other.

The CPU 206 comprises a processor, such as one or more conventional microprocessors and one or more supplementary co-processors such as math co-processors for offloading workload from the CPU 206. The CPU 206 is in communication with the communications interface 208 and the input/output controller 210, through which the CPU 206 communicates with other devices such as other servers, user terminals, or devices. The communications interface 208 and the input/output controller 210 may include multiple communication channels for simultaneous communication with, for example, other processors, servers or client terminals.

The CPU 206 is also in communication with the data storage device 211 and system memory 201. The data storage device 211 and system memory 201 may comprise an appropriate combination of magnetic, optical or semiconductor memory, and may include, for example, RAM 202, ROM 204, flash drive, an optical disc such as a compact disc or a hard disk or drive. The system memory 201 may be any suitable combination of fixed and/or removable memory, and may include any suitable combination of volatile or non-volatile storage. The memory 201 may be physically located inside a user device or server or may be physically located outside of the user device (e.g., as part of cloud-based storage) and accessed by the user device over a communications network. The CPU 206 and the data storage device each may be, for example, located entirely within a single computer or other computing device; or connected to each other by a communication medium, such as a USB port, serial port cable, a coaxial cable, an Ethernet cable, a telephone line, a radio frequency transceiver or other similar wireless or wired medium or combination of the foregoing. For example, the CPU 206 may be connected to the data storage device via the communications interface 208. The CPU 206 may be configured to perform one or more particular processing functions.

The data storage device 211 may store, for example, (i) an operating system 212 for the computing device 200; (ii) one or more applications 214 (e.g., computer program code or a computer program product) adapted to direct the CPU 206 in accordance with the systems and methods described here, and particularly in accordance with the processes described in detail with regard to the CPU 206; and/or (iii) database(s) 216 adapted to store information that may be utilized by the program.

The operating system 212 and applications 214 may be stored, for example, in a compressed, an uncompiled and an encrypted format, and may include computer program code. The instructions of the program may be read into a main memory of the processing circuitry from a computer-readable medium other than the data storage device, such as from the ROM 204 or from the RAM 202. While execution of sequences of instructions in the program causes the CPU 206 to perform the process steps described herein, hard-wired circuitry may be used in place of, or in combination with, software instructions for implementation of the processes of systems and methods described in this application. Thus, the systems and methods described are not limited to any specific combination of hardware and software.

Suitable computer program code may be provided for performing one or more functions as described herein. The program also may include program elements such as an operating system 212, a database management system and "device drivers" that allow the processing circuitry to interface with computer peripheral devices (*e.g*., a video display, a keyboard, a computer mouse, *etc.*) via the input/output controller 210.

The term "computer-readable medium" as used herein refers to any non-transitory medium that provides or participates in providing instructions to the processing circuitry of the computing device 200 (or any other processing circuitry of a device described herein) for execution. Such a medium may take many forms, including but not limited to, non-volatile media and volatile media. Non-volatile media include, for example, optical, magnetic, or optomagnetic disks, or integrated circuit memory, such as flash memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM or EEPROM (electronically erasable programmable read-only memory), a FLASH-EEPROM, any other memory chip or cartridge, or any other non-transitory medium from which a computer can read.

Various forms of computer readable media may be involved in carrying one or more sequences of one or more instructions to the CPU 206 (or any other processing circuitry of a device described herein) for execution. For example, the instructions may initially be borne on a magnetic disk of a remote computer (not shown). The remote computer can load the instructions into its dynamic memory and send the instructions over an Ethernet connection, cable line, or even telephone line using a modem. A communications device local to a computing device 200 (e.g., a server) can receive the data on the respective communications line and place the data on a system bus for the processor. The system bus carries the data to main memory, from which the processing circuitry retrieves and executes the instructions. The instructions received by main memory may optionally be stored in memory either before or after execution by the processor. In addition, instructions may be received via a communication port as electrical, electromagnetic or optical signals, which are exemplary forms of wireless communications or data streams that carry various types of information.

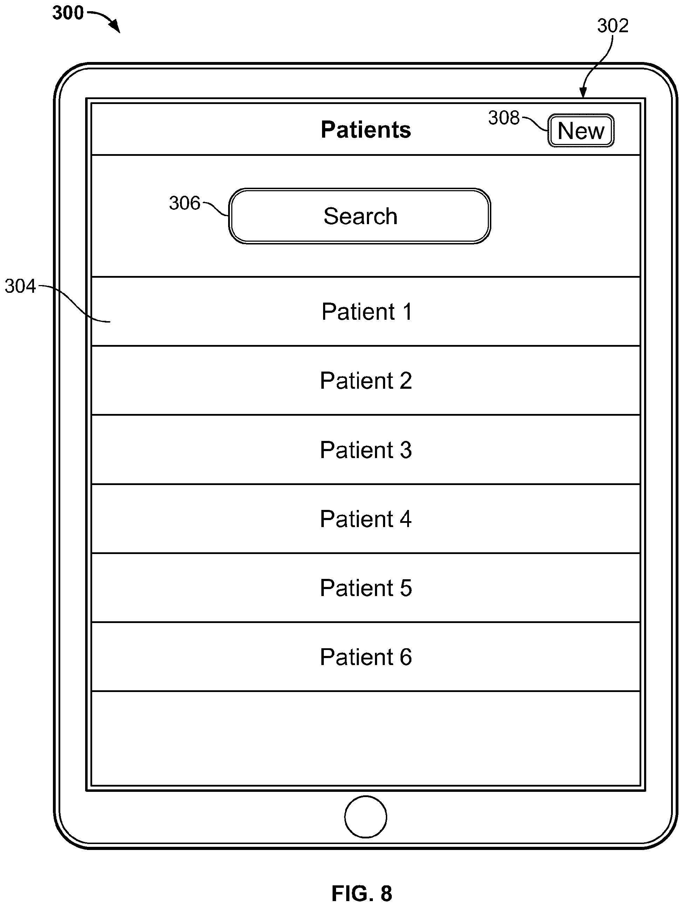

The implementation of the methods and systems discussed above provides prompt and accurate wound status and progression feedback to a patient or physician for monitoring or adjusting wound care. Whether the image processing, pH calculation, and data storage is provided locally at a user's device or remotely at one or more server or cloud components, a local device provides an interface to the user for providing and receiving data and information that is used in or results from such processing and data storage. The series of screenshots discussed below and shown in Figures 8-13 demonstrate data and information that may be transmitted to or received from a user, either on a local user device or on an accessory device in communication with a user device. It will be understood that the screens, fields, and data shown in the displays in Figures 8-13 may be modified or omitted as desired, and the display descriptions below do not limit or exclude the displays and data that may be relayed to a user.

Figure 8 shows an illustrative display 302 on a user device 300 for identifying patients and receiving user selections of a patient. Such selection may be used, for example, to identify a particular patient being monitored for current pH value readings from a plurality of patients who use the same device 300 or who visit the same doctor who uses the device 300 for patient monitoring. The display 302 facilitates identification and tracking of the patients in such situations when multiple records are accessed from the same device. Depending on the system in which the user device 300 is implemented, the list of patients may provide access to locally-stored data and information specific to each patient, or may provide access to data and information stored locally. In response to user selection of a patient when data is stored locally, the user device 300 accesses the locally stored data to display to the user or to update a stored record with a new pH reading. In response to user selection of a patient when data is stored remotely, the user device 300 transmits a request to the remote storage device to send the data for user display or transmits a new pH reading to be added to the remotely-stored record.

Wound monitoring systems may also provide automated patient identification that determines the patient from the wound dressing image. A wound dressing may include a name, number, barcode, QR code, or other unique identifier that is related with a particular patient. The identifier may be included on a wound dressing, such as dressing, 150 in Figure 4, and a box of bandages can all include the same identifier. Alternatively, the identifier may be provided separate from a dressing, for example, as a sticker, and the patient or physician may transfer the identifier to a new dressing each time the wound bandage is changed. When the bandage and identifier are imaged for the first time, the device alerts the user that the identifier has not been associated with a patient and provides the display 302 for the user to select the patient. On subsequent readings, the patient is automatically determined from the identifier, and the display 302 is bypassed.

The screen 302 includes a list of patients each identified by name, for example "Patient 1" for identifier 304 in the list. From this list, a user can select one of the identifiers to access records for the patient, begin a new wound pH reading for the patient, view or edit identifying information for the patient, or perform any other desired function for that particular patient. In situations where the list of patients is quite long, a search query box 306 may be included to facilitate patient selection without requiring a user to scroll through the long list of patients to find the desired identifier. The list of patient identifiers may automatically update in real time as a user types in the query box 306 to filter the list and eliminate any patient identifiers that do not contain the query being entered.

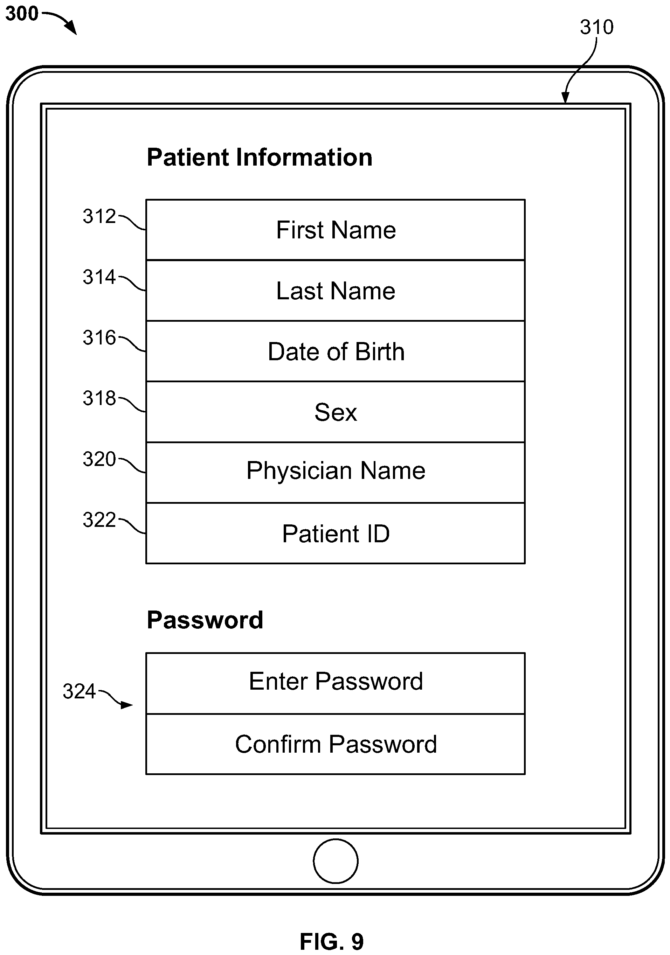

If a new patient is being monitored, the patient or the patient's physician may select the new patient option 308 to add a new record to the database of stored patient information and data. When a user selects the new patient option 308, a prompt screen is displayed to the user for identification information to be used for the new patient's record. The display 310 on user device 300 shown in Figure 9 depicts an illustrative example of such a new patient prompt screen. The display 310 includes fields for patient identification and information that can be used to identify the particular patient and associate any records kept from pH monitoring with the particular patient to which they relate. While the display 310 includes particular information fields, some of the fields shown may be omitted, or additional information fields may be included in the display 310, depending on the information desired for patient record keeping in a particular implementation.

The display 310 includes a first name field 312, last name field 314, date of birth field 316, and sex field 318 for identifying the specific patient. These standard identifying fields can be stored in patient records for each patient in a given system, and can be used to sort the patients or filter the patients as desired. The first name field 312 and last name field 314 in particular can be used to efficiently identify or find patients, for example by searching for patients in a list of records using a search like the search query box 306 shown in Figure 8.

The display 310 also includes a physician name field 320 and a patient ID field 322 that may be used to connect physicians and patients in certain implementations. The physician name field 320 may be used, for example, in systems that store patient records for many patients and for patients of different physicians. For example, a cloud computing system may be maintained by a provider of the pH monitoring application running on user device 300, or by the provider of wound dressings used to monitor pH. The cloud system may use the physician field 320 to group patient records by physician and provide a given physician with access to the records for only his or her patients. In such implementations, the patient ID field 322 may be used to facilitate record keeping for physicians. Each physician may assign ID numbers to each patient for easier record keeping, and the pH monitoring readings for each patient may be stored by ID number in addition to patient name. For security purposes, the display 310 may also include password fields 324 for setting and confirming a password for each new patient that is later required to access any records or take new readings for the particular patient.

The pH readings that are stored for individual patients are obtained from images captured by the user device 300. The device 300, preferably through an application running on the device, provides a user with image capture cues to facilitate capturing quality images that will result in reliable pH readings for a wound dressing. Display 326 on user device 300 in Figure 10 shows an embodiment of such an image capture screen. The display 326 includes a patient identifier 328, image capture screen 330, and tabs menu 340. The patient identifier 328 displays a selected patient for whom a reading is being taken for implementations in which the patient is selected before pH readings are obtained. In implementations where images and pH readings can be obtained and the associated patient identified after the readings, the patient identifier 328 may be blank or may be omitted from the display 326.

The tabs menu 340 facilitates navigation between the various screens provided on user device 300, and may be included or omitted from any of the displays discussed herein as desired. The menu 340 includes a patients tab 342 for accessing a list of patients for whom records are stored and accessible, a pH reading tab 344 to access the image capture display 326 and take readings, a trend tab 346 to access a list or graph of past readings for a particular patient, and a settings tab 348 to access general configurable application and image capture settings.

The image capture screen 330 of display 326 helps a user take quality images of wound dressings, for example dressing 336 in Figure 10, to provide reliable and repeatable pH readings. The screen 330 displays a guiding frame 332 for a user to indicate the optimal orientation of dressing 336 for image capture. The guiding frame 332 gives the user a visual cue to align the dressing 336 before causing the user device 300 to capture an image of the dressing 336 for pH reading. In addition to providing the guiding frame 332, the user device 300 may monitor the orientation of the dressing 336, for example by detecting the location of corner indicators 350 or calibration strip indicators 352, and automatically capture an image for processing when the dressing 336 is adequately aligned with the guiding frame 332. Once the pH indicator 334, color calibration strip 338, corner indicators 350, and calibration strip indicators 352 are all positioned within the guiding frame 332, the user is provided with visual confirmation that a suitable image of the dressing 336 can be captured, whether done manually by pressing a capture button or automatically by the user device 300.

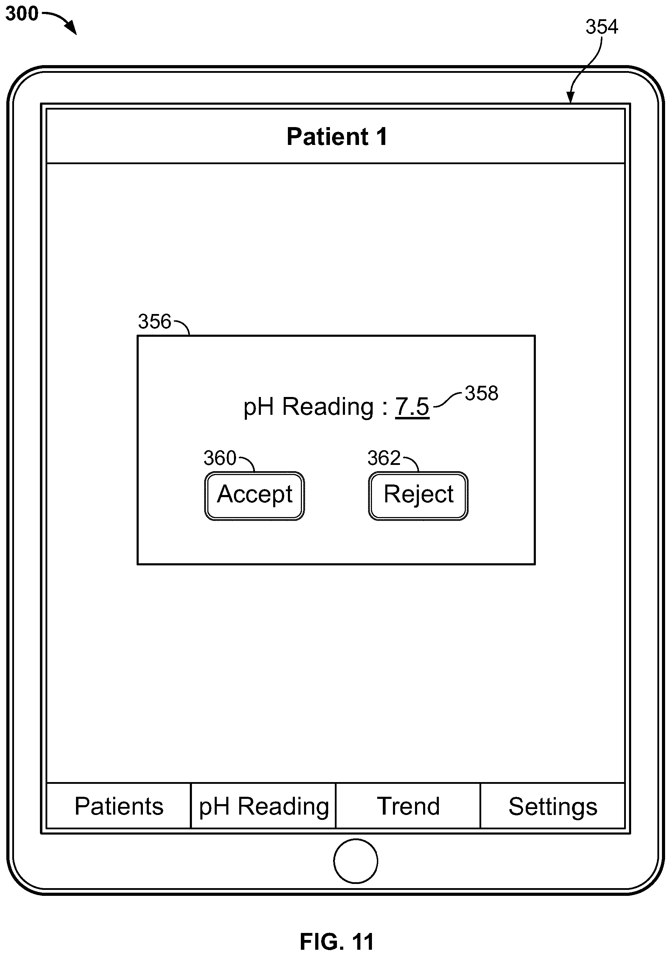

An image that is captured in the image capture screen 330 is processed, either locally at user device 300 or remotely at a processor in communication with user device 300 over a network, to return a calculated pH value for the wound dressing 336. Display 354 in Figure 11 shows an illustrative screen that displays the calculated value to a patient or physician. The display 354 includes a reading window 356 that provides the user with the calculated pH 358 for the dressing image that was captured. The window 356 also presents the user with an accept option 360 and a reject option 362 that a user can use to indicate whether or not the calculated pH 358 is reasonable or acceptable. If a user determines that the calculated pH358 is reasonable, he or she may select the accept option 360, and the calculated pH 358 can be added to the patient's record. If a user determines that the calculated pH 358 is not reasonable, for example if it is nonsensical or seems either inconsistent or too high or low, the user can select the reject option 362. If the reject option 362 is selected, the calculated pH 358 may not be stored in a patient's record, or may be stored in the record with a special flag to indicate the calculated pH 358 was marked as unreliable or wrong. Optionally, the user device 300 may provide the user with a prompt or additional option to capture another dressing image if the reject option 362 is selected.

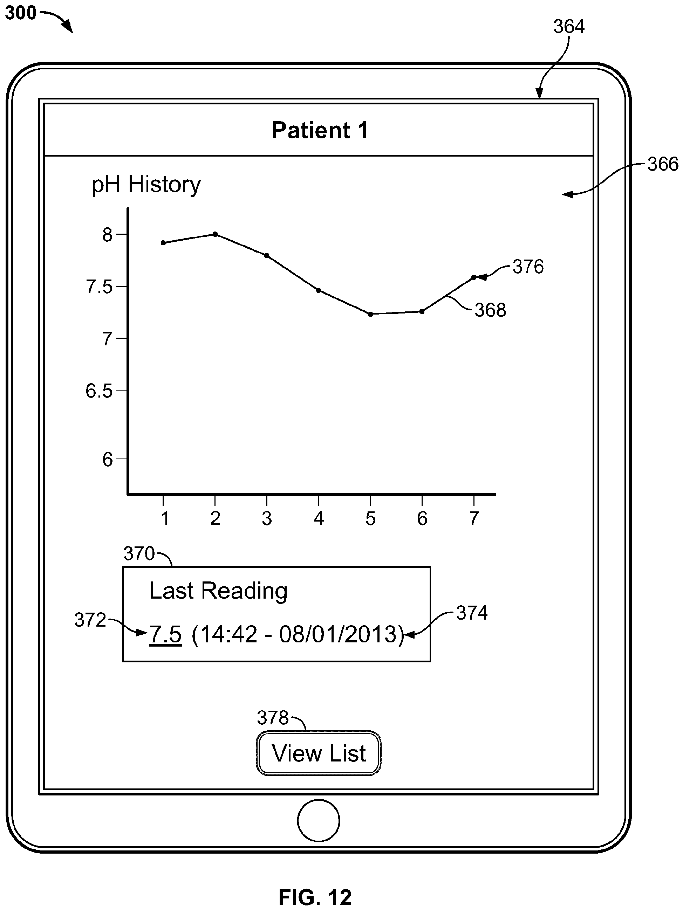

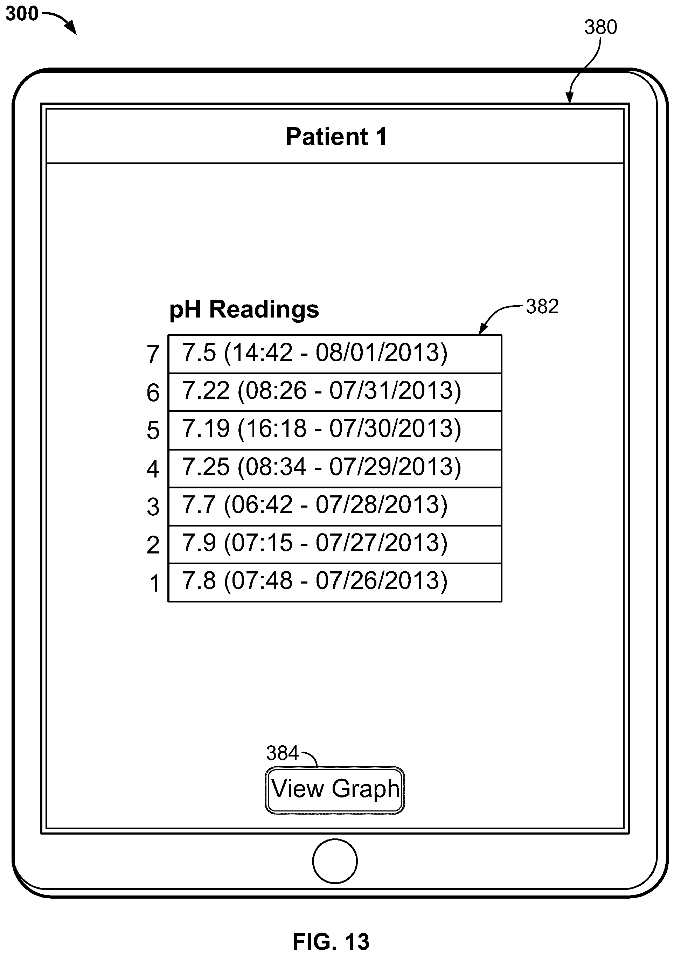

The user device 300 also provides a user with screens that allow a patient or physician to review patient data either after a reading is accepted from display 354 in Figure 11, after a patient is selected from display 302 in Figure 8, after trend tab 346 is selected from display 326, or when patient records are accessed from any other screens. The displays of user device 300 shown in Figures 12 and 13 show screens that may be implemented to provide a patient or physician with a data record review interface.

Figure 12 shows a display 364 on user device 300 that provides a graphical representation of pH reading history for a particular patient. The display 364 includes a graph 366 showing a trend 368 in calculated pH values over time for an identified patient "Patient 1." The trend 368 plots the last seven pH values that were obtained for the patient in the graph 366. More or fewer than seven pH values may be displayed in the graph 366, and the size and spacing of the graph may be scaled as appropriate for showing more of fewer pH data points.

The display 364 also shows a last reading window 370 that identifies the last calculated pH value 372 to the user. This calculated pH value 372 corresponds to the last point 376 plotted in trend 368 of graph 366. The window 370 also identifies the date and time 374 at which the calculated pH value 372 was obtained for the patient At the bottom of display 364, a list option 378 is provided. If a user selects the list option 378, the user device 364 displays a list of all of the calculated pH values plotted in the graph 366, along with date and time information and any other desired identification information for each point.

Figure 13 shows a list display 380 that may be displayed in response to a user selection of the list option 378 of display 364. The display 380 includes a data list 382 that shows the calculated pH value and corresponding date and time for each data point plotted in the graph 366 of Figure 12. While seven data points are shown in the list 382, any number of data points can be displayed, and the list 382 can be scaled accordingly to fit the desired number of points. If there are too many points to fit on the display 380, the list 382 may be scrollable to allow the user to go backwards and forwards through the serial data points. In addition to the list 382, the display 380 includes a view graph option 384 that allows the user to toggle the user device 300 to a graphical display of the data in the list 382, for example by returning to the screen 364 of Figure 12 if the graph option 384 is selected.
The scope of the invention is defined by the system of independent claim 1. Optional features are defined in the dependent claims.

The foregoing is merely illustrative of the principles of the disclosure, and the systems, devices, and methods can be practiced by other than the described embodiments, which are presented for purposes of illustration and not of limitation. It is to be understood that the systems, devices, and methods disclosed herein, while shown for use in wound monitoring approaches using wound dressing having color pH indicators, user devices, and servers, may be applied to systems, devices, and methods to be used in other approaches for wound monitoring using pH tracking or tracking of other wound indicators using color bandages.

## Claims

1. A system for monitoring a wound, comprising:
means for capturing an image of a wound dressing;
means for determining the color of a pH indicator on the wound dressing, wherein the means for determining the color comprises means for extracting RGB values from the captured image;
means for calculating a pH value for the wound dressing from the dressing RGB values; and
means for displaying an indication of the calculated pH value;
wherein means for extracting dressing RGB values from the captured image comprises means for determining individual pixel RGB values for each one of a plurality of pixels in the image and means for averaging the individual pixel RGB values for the plurality of pixels to determine the dressing RGB values; and
further comprising means for capturing an image of a color calibration strip wherein the color calibration strip is captured in the same image as the wound dressing and further comprising means for extracting calibration RGB values from the image of the color calibration strip for each of a plurality of color blocks in the color calibration strip wherein each color block is associated with a standardized pH value; and
wherein the pH value for the wound dressing is calculated using the dressing RGB values and the calibration RGB values.

2. The system of claim 1, further comprising means for displaying a guiding frame during image capture, wherein the guiding frame provides an indication of proper wound dressing alignment to a user and optionally further comprising means for detecting the alignment of the wound dressing relative to the displayed guiding frame, wherein the image is automatically captured by the means for capturing when the wound dressing is properly aligned with the guiding frame.

3. The system of any of the preceding claims, further comprising:
means for rejecting an image having inadequate light or excessive shadow; and
means for displaying a request to a user to capture a new image.

4. The system of any of the preceding claims, further comprising means for displaying an option to accept or reject the calculated pH value when the calculated pH value is displayed.

5. The system of any of the preceding claims, further comprising means for storing the calculated pH value in a record of pH values.

6. The system of claim 5, further comprising means for receiving user input identifying a particular patient, wherein the stored record is associated with the particular patient and wherein the user input comprises a selection of the particular patient from a list of stored patients or wherein the user input comprises identification information for a new patient.

7. The system of any of claims 5-6, further comprising means for displaying a trend of pH values for the particular patient and optionally wherein the displayed trend comprises at least one of a graph and a list of pH values.

8. The system of any of the preceding claims, further comprising means for defining a center point of the captured image.

9. The system of claim 8, further comprising means for defining a dressing circle region around the center point of the captured image, wherein the dressing circle region comprises the plurality of pixels for which the individual pixel RGB values are determined
and wherein the dressing circle region has a radius between 5 and 100 pixels, preferably between 10 and 50 pixels and most preferably wherein the dressing circle region has a radius between 20 and 30 pixels.

10. The system of any of claims 1-9, wherein the means for extracting calibration RGB values for each of the plurality of color blocks comprises means for determining individual pixel RGB values for each one of a plurality of pixels in a color block and means for averaging the individual pixel RGB values for the plurality of pixels in the color block to determine the calibration RGB values for the color block.

11. The system of claim 10, further comprising means for defining a center point of each of the plurality of color blocks wherein the center points are defined from alignment indicators positioned on either side of the color calibration strip, optionally further comprising means for defining a calibration circle region around the center point of each color block, wherein each of the calibration circle regions comprises the plurality of pixels for which the individual pixel RGB values are determined in each color block and optionally wherein each of the calibration circle regions has a radius between 3 and 10 pixels and wherein each of the calibration circle regions has a radius of 5 pixels.

12. The system of any of claims 1 to 11, further comprising means for calculating a distance between the dressing RGB values and each of the calibration RGB values in a three-dimensional space, optionally further comprising:
means for determining the two smallest calculated distances; and
means for calculating the pH value for the wound dressing based on the RGB calibration values and standardized pH values associated with the two smallest distances and optionally further comprising means of normalizing the dressing RGB values to a line defined by the two RGB calibrations values associated with the two shortest distances; and
means for calculating the pH value for the wound dressing from the normalized position of the dressing RGB values on the line.

## Patentansprüche

1. Ein System zum Überwachen einer Wunde, beinhaltend:
ein Mittel zum Erfassen eines Bilds eines Wundverbands;
ein Mittel zum Bestimmen der Farbe eines pH-Indikators auf dem Wundverband, wobei das Mittel zum Bestimmen der Farbe ein Mittel zum Extrahieren von RGB-Werten aus dem erfassten Bild beinhaltet;
ein Mittel zum Berechnen eines pH-Werts für den Wundverband aus den Verband-RGB-Werten; und
ein Mittel zum Anzeigen einer Angabe des berechneten pH-Werts;
wobei das Mittel zum Extrahieren von Verband-RGB-Werten aus dem erfassten Bild ein Mittel zum Bestimmen der RGB-Werte individueller Pixel für jedes einzelne einer Vielzahl von Pixeln in dem Bild und ein Mittel zum Mitteln der RGB-Werte individueller Pixel für die Vielzahl von Pixeln zum Bestimmen der Verband-RGB-Werte beinhaltet; und
ferner beinhaltend ein Mittel zum Erfassen eines Bilds eines Farbkalibrierungsstreifens, wobei der Farbkalibrierungsstreifen in demselben Bild wie der Wundverband erfasst wird, und ferner beinhaltend ein Mittel zum Extrahieren von Kalibrierungs-RGB-Werten aus dem Bild des Farbkalibrierungsstreifens für jeden von einer Vielzahl von Farbblöcken in dem Farbkalibrierungsstreifen, wobei jeder Farbblock mit einem standardisierten pH-Wert assoziiert ist; und
wobei der pH-Wert für den Wundverband unter Verwendung der Verband-RGB-Werte und der Kalibrierungs-RGB-Werte berechnet wird.

2. System gemäß Anspruch 1, ferner beinhaltend ein Mittel zum Anzeigen eines Führungsrahmens während der Bilderfassung, wobei der Führungsrahmen einem Benutzer eine Angabe der korrekten Ausrichtung des Wundverbands bereitstellt, und optional ferner beinhaltend ein Mittel zum Detektieren der Ausrichtung des Wundverbands relativ zu dem angezeigten Führungsrahmen, wobei das Bild von dem Mittel zum Erfassen automatisch erfasst wird, wenn der Wundverband korrekt nach dem Führungsrahmen ausgerichtet ist.

3. System gemäß einem der vorhergehenden Ansprüche, ferner beinhaltend:
ein Mittel zum Ablehnen eines Bilds mit unzureichendem Licht oder übermäßigem Schatten; und
ein Mittel zum Anzeigen einer Aufforderung an einen Benutzer, ein neues Bild zu erfassen.

4. System gemäß einem der vorhergehenden Ansprüche, ferner beinhaltend ein Mittel zum Anzeigen einer Option, den berechneten pH-Wert zu akzeptieren oder abzulehnen, wenn der berechnete pH-Wert angezeigt wird.

5. System gemäß einem der vorhergehenden Ansprüche, ferner beinhaltend ein Mittel zum Speichern des berechneten pH-Werts in einer Aufzeichnung von pH-Werten.

6. System gemäß Anspruch 5, ferner beinhaltend ein Mittel zum Empfangen einer Benutzereingabe, die einen bestimmten Patienten identifiziert, wobei die gespeicherte Aufzeichnung mit dem bestimmten Patienten assoziiert ist und wobei die Benutzereingabe eine Auswahl des bestimmten Patienten aus einer Liste von gespeicherten Patienten beinhaltet oder wobei die Benutzereingabe Identifizierungsinformationen für einen neuen Patienten beinhaltet.

7. System gemäß einem der Ansprüche 5-6, ferner beinhaltend ein Mittel zum Anzeigen eines Trends der pH-Werte für den bestimmten Patienten, und wobei optional der angezeigte Trend mindestens eines von einem Graphen und einer Liste von pH-Werten beinhaltet.

8. System gemäß einem der vorhergehenden Ansprüche, ferner beinhaltend ein Mittel zum Definieren eines Mittelpunkts des erfassten Bilds.

9. System gemäß Anspruch 8, ferner beinhaltend ein Mittel zum Definieren eines Verbandkreisbereichs um den Mittelpunkt des erfassten Bilds, wobei der Verbandkreisbereich die Vielzahl von Pixeln beinhaltet, für die die RGB-Werte individueller Pixel bestimmt werden,
und wobei der Verbandkreisbereich einen Radius von zwischen 5 und 100 Pixeln, vorzugsweise zwischen 10 und 50 Pixeln aufweist, und wobei am bevorzugtesten der Verbandkreisbereich einen Radius von zwischen 20 und 30 Pixeln aufweist.

10. System gemäß einem der Ansprüche 1-9, wobei das Mittel zum Extrahieren von Kalibrierungs-RGB-Werten für jeden der Vielzahl von Farbblöcken ein Mittel zum Bestimmen der RGB-Werte individueller Pixel für jedes einzelne einer Vielzahl von Pixeln in einem Farbblock und ein Mittel zum Mitteln der RGB-Werte individueller Pixel für die Vielzahl von Pixeln in dem Farbblock zum Bestimmen der Kalibrierungs-RGB-Werte für den Farbblock beinhaltet.

11. System gemäß Anspruch 10, ferner beinhaltend ein Mittel zum Definieren eines Mittelpunkts von jedem der Vielzahl von Farbblöcken, wobei die Mittelpunkte anhand Ausrichtungsindikatoren, die auf beiden Seiten des Farbkalibrierungsstreifens positioniert sind, definiert werden, optional ferner beinhaltend ein Mittel zum Definieren eines Kalibrierungskreisbereichs um den Mittelpunkt jedes Farbblocks, wobei jeder der Kalibrierungskreisbereiche die Vielzahl von Pixeln beinhaltet, für die die RGB-Werte individueller Pixel in jedem Farbblock bestimmt werden, und wobei optional jeder der Kalibrierungskreisbereiche einen Radius von zwischen 3 und 10 Pixeln aufweist und wobei jeder der Kalibrierungskreisbereiche einen Radius von 5 Pixeln aufweist.

12. System gemäß einem der Ansprüche 1 bis 11, ferner beinhaltend ein Mittel zum Berechnen einer Entfernung zwischen den Verband-RGB-Werten und jedem der Kalibrierungs-RGB-Werte in einem dreidimensionalen Raum, optional ferner beinhaltend:
ein Mittel zum Bestimmen der zwei geringsten berechneten Entfernungen; und
ein Mittel zum Berechnen des pH-Werts für den Wundverband basierend auf den RGB-Kalibrierungswerten und standardisierten pH-Werten, die mit den zwei geringsten Entfernungen assoziiert sind, und optional ferner beinhaltend ein Mittel zum Normieren der Verband-RGB-Werte auf eine Linie, die von den zwei RGB-Kalibrierungswerten definiert wird, die mit den zwei geringsten Entfernungen assoziiert sind; und
ein Mittel zum Berechnen des pH-Werts für den Wundverband aus der normierten Position der Verband-RGB-Werte auf der Linie.

## Revendications

1. Un système destiné à surveiller une plaie, comprenant :
un moyen destiné à capturer une image d'un pansement pour plaie ;
un moyen destiné à déterminer la couleur d'un indicateur de pH sur le pansement pour plaie, où le moyen destiné à déterminer la couleur comprend un moyen destiné à extraire des valeurs RVB de l'image capturée ;
un moyen destiné à calculer une valeur de pH pour le pansement pour plaie à partir des valeurs RVB de pansement ; et
un moyen destiné à afficher une indication de la valeur de pH calculée ;
où un moyen destiné à extraire des valeurs RVB de pansement de l'image capturée comprend un moyen destiné à déterminer des valeurs RVB de pixels individuels pour chaque pixel d'une pluralité de pixels dans l'image et un moyen destiné à faire une moyenne des valeurs RVB de pixels individuels pour la pluralité de pixels afin de déterminer les valeurs RVB de pansement ; et
comprenant en outre un moyen destiné à capturer une image d'une bandelette d'étalonnage de couleur où la bandelette d'étalonnage de couleur est capturée dans la même image que le pansement pour plaie et comprenant en outre un moyen destiné à extraire des valeurs RVB d'étalonnage de l'image de la bandelette d'étalonnage de couleur pour chaque bloc d'une pluralité de blocs de couleur dans la bandelette d'étalonnage de couleur où chaque bloc de couleur est associé à une valeur de pH normalisée ; et
où la valeur de pH pour le pansement pour plaie est calculée à l'aide des valeurs RVB de pansement et des valeurs RVB d'étalonnage.

2. Le système de la revendication 1, comprenant en outre un moyen destiné à afficher un cadre de guidage pendant la capture d'image, où le cadre de guidage apporte une indication d'un alignement convenable de pansement pour plaie à un utilisateur et facultativement comprenant en outre un moyen destiné à détecter l'alignement du pansement pour plaie relativement au cadre de guidage affiché, où l'image est capturée automatiquement par le moyen destiné à capturer lorsque le pansement pour plaie est aligné convenablement avec le cadre de guidage.

3. Le système de n'importe lesquelles des revendications précédentes, comprenant en outre :
un moyen destiné à rejeter une image ayant une lumière inadéquate ou une ombre excessive ; et
un moyen destiné à afficher une demande à un utilisateur de capturer une nouvelle image.

4. Le système de n'importe lesquelles des revendications précédentes, comprenant en outre un moyen destiné à afficher une option visant à accepter ou rejeter la valeur de pH calculée lorsque la valeur de pH calculée est affichée.

5. Le système de n'importe lesquelles des revendications précédentes, comprenant en outre un moyen destiné à stocker la valeur de pH calculée dans un registre de valeurs de pH.

6. Le système de la revendication 5, comprenant en outre un moyen destiné à recevoir une entrée utilisateur identifiant un patient particulier, où le registre stocké est associé au patient particulier et où l'entrée utilisateur comprend une sélection du patient particulier dans une liste de patients stockés ou bien où l'entrée utilisateur comprend des informations d'identification pour un nouveau patient.

7. Le système de n'importe lesquelles des revendications 5 à 6, comprenant en outre un moyen destiné à afficher une tendance de valeurs de pH pour le patient particulier et facultativement où la tendance affichée comprend au moins un élément parmi un graphe et une liste de valeurs de pH.

8. Le système de n'importe lesquelles des revendications précédentes, comprenant en outre un moyen destiné à définir un point central de l'image capturée.

9. Le système de la revendication 8, comprenant en outre un moyen destiné à définir une région circulaire de pansement autour du point central de l'image capturée, où la région circulaire de pansement comprend la pluralité de pixels pour lesquels les valeurs RVB de pixels individuels sont déterminées
et où la région circulaire de pansement a un rayon compris entre 5 et 100 pixels, de préférence entre 10 et 50 pixels et de préférence encore où la région circulaire de pansement a un rayon compris entre 20 et 30 pixels.

10. Le système de n'importe lesquelles des revendications 1 à 9, où le moyen destiné à extraire des valeurs RVB d'étalonnage pour chaque bloc de la pluralité de blocs de couleur comprend un moyen destiné à déterminer des valeurs RVB de pixels individuels pour chaque pixel d'une pluralité de pixels dans un bloc de couleur et un moyen destiné à faire une moyenne des valeurs RVB de pixels individuels pour la pluralité de pixels dans le bloc de couleur afin de déterminer les valeurs RVB d'étalonnage pour le bloc de couleur.

11. Le système de la revendication 10, comprenant en outre un moyen destiné à définir un point central de chaque bloc de la pluralité de blocs de couleur où les points centraux sont définis à partir d'indicateurs d'alignement positionnés de part et d'autre de la bandelette d'étalonnage de couleur, facultativement comprenant en outre un moyen destiné à définir une région circulaire d'étalonnage autour du point central de chaque bloc de couleur, où chaque région des régions circulaires d'étalonnage comprend la pluralité de pixels pour lesquels les valeurs RVB de pixels individuels sont déterminées dans chaque bloc de couleur et facultativement où chaque région des régions circulaires d'étalonnage a un rayon compris entre 3 et 10 pixels et où chaque région des régions circulaires d'étalonnage a un rayon de 5 pixels.

12. Le système de n'importe lesquelles des revendications 1 à 11, comprenant en outre un moyen destiné à calculer une distance entre les valeurs RVB de pansement et chaque valeur des valeurs RVB d'étalonnage dans un espace tridimensionnel, facultativement comprenant en outre :
un moyen destiné à déterminer les deux distances calculées les plus courtes ; et
un moyen destiné à calculer la valeur de pH pour le pansement pour plaie sur la base des valeurs d'étalonnage RVB et des valeurs de pH normalisées associées aux deux distances le plus courtes et facultativement comprenant en outre un moyen de normalisation des valeurs RVB de pansement par rapport à une ligne définie par les deux valeurs d'étalonnage RVB associées aux deux distances les plus courtes ; et
un moyen destiné à calculer la valeur de pH pour le pansement pour plaie à partir de la position normalisée des valeurs RVB de pansement sur la ligne.
